# DEMANDE DE BREVET EUROPEEN

(11) **EP 0 556 123 A1**
(43) Date de publication de la demande: **18.08.1993**
(21) Numéro de dépôt: 93400348.4
(22) Date de dépôt: 11.02.1993
(51) Int. Cl.: C07D 277/24, C07D 277/34, C07D 277/36, C07D 277/32, C07D 413/04, A01N 53/00

(54) **Nouveaux esters pyréthrinoides dérivés d'alcools thiazoliques, leur procédé de préparation et leur application comme pesticides**

(30) Priorité: 12.02.1992 FR 9201556
(71) Demandeur: ROUSSEL UCLAF, F-93230 Romainville (FR)
(72) Inventeur: Benoit, Marc, F-13360 Roquevaire (FR); Demoute, Jean-Pierre, F-93360 Neuilly Plaisance (FR); Wehrey, Christian, F-94120 Fontenay sous Bois (FR)
(74) Mandataire: Vieillefosse, Jean-Claude

(57) **Abrégé**

L'invention a pour objet les composés de formule (I) : 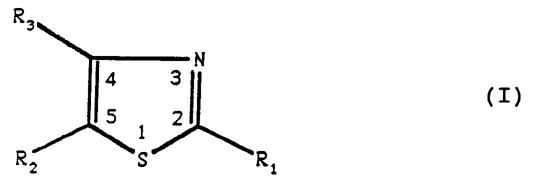
dans laquelle l'un des radicaux R₁, R₂ et R₃ représente un radical 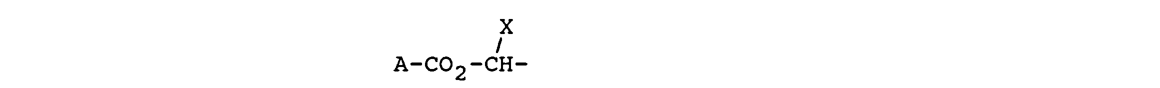
dans lequel X représente un atome d'hydrogène, un radical C≡N, un radical alkyle, alkényle ou alkynyle, A représente le reste d'un acide pyréthrinoïde et les deux radicaux choisis parmi les radicaux R₁, R₂ et R₃ qui ne représentent pas un radical : 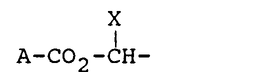
sont identiques ou différents et représentent ou un atome d'hydrogène ou un atome d'halogène ou un radical alkyle, alkényle ou alkynyle éventuellement substitué ou un radical hydroxyle, O-alkyle, O-alkényle ou O-alkynyle, CO₂-alkyle, CO₂-alkényle, CO₂-alkynyle, S(O)ₙ-alkyle, S(O)ₙ-alkényle ou S(O)ₙ-alkynyle, n représentant le nombre 0, 1 ou 2, éventuellement substitué ou un radical aryle, O-aryle ou thioaryle éventuellement substitué ou un radical hétéroaryle ou hétéroaryloxy ou un radical C≡N, NH₂ ou NO₂.

Les composés de formule (I) présentent d'intéressantes propriétés pesticides.

## Description

La présente invention concerne de nouveaux esters pyréthrinoïdes dérivés d'alcools thiazoliques, leur procédé de préparation et leur application comme pesticides.

L'invention a pour objet sous toutes leurs formes stéréoisomères possibles, ainsi que leurs mélanges, les composés de formule (I) : 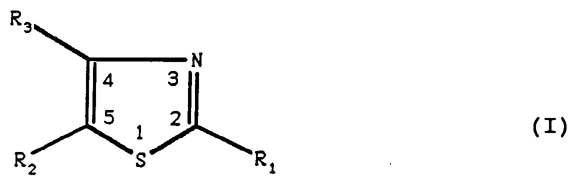
dans laquelle l'un des radicaux R₁, R₂ ou R₃ représente un radical : 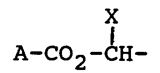
dans lequel :
X représente un atome d'hydrogène, un radical C≡N, un radical alkyle, alkényle ou alkynyle renfermant jusqu'à 4 atomes de carbone et
ou bien A représente un radical : 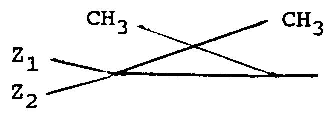
dans lequel Z₁ représente un atome d'hydrogène et
- soit Z₂ représente un radical : 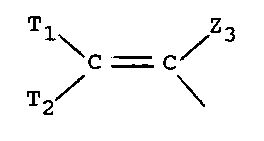 dans lequel Z₃ représente un atome d'hydrogène ou d'halogène et T₁ représente un atome d'hydrogène, un atome d'halogène, un radical alkyloxy ou alkyle renfermant de 1 à 8 atomes de carbone éventuellement substitués par des halogènes, un radical mono-, di- ou trifluorométhyle ou cyano ou un noyau phényle éventuellement substitué par un halogène et T₂ représente un atome d'hydrogène, un atome d'halogène, un radical alkyloxy renfermant de 1 à 8 atomes de carbone éventuellement substitué par des halogènes ou un radical alkyle renfermant de 1 à 8 atomes de carbone substitués par des halogènes, un radical mono-, di- ou trifluorométhyle ou cyano ou un noyau phényle éventuellement substitué par un halogène ou T₁ et T₂ forment ensemble un radical cycloalkyle renfermant de 3 à 6 atomes de carbone ou un radical : 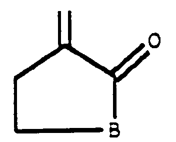 dans lequel B représente un atome d'oxygène ou de soufre ;
- soit Z₂ représente un radical : 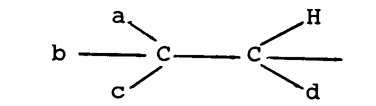 dans lequel a, b, c et d, identiques ou différents représentent chacun un atome d'halogène,
- soit Z₂ représente un radical : 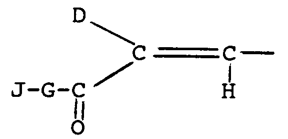 dans lequel D représente un atome d'hydrogène ou d'halogène, un radical alkyloxy renfermant de 1 à 8 atomes de carbone, G représente un atome d'oxygène ou de soufre et J représente ou bien un radical alkyle linéaire, ramifié ou cyclique, saturé ou insaturé, renfermant de 1 à 8 atomes de carbone, éventuellement substitué par un ou plusieurs groupements fonctionnels, identiques ou différents, ou bien un groupement aryle renfermant de 6 à 14 atomes de carbone, éventuellement substitué par un ou plusieurs groupements fonctionnels identiques ou différents, ou bien un radical hétérocyclique éventuellement substitué par un ou plusieurs groupements fonctionnels, identiques ou différents,
   ou bien A représente un radical : 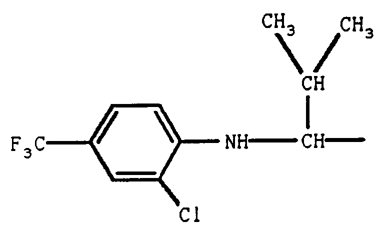 ou un radical : 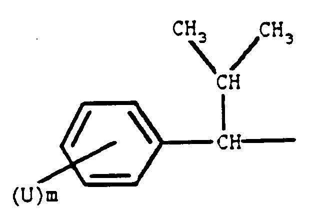 dans lequel U, en position quelconque sur le noyau benzénique, représente un atome d'halogène, un radical alkyle renfermant de 1 à 8 atomes de carbone ou un radical alcoxy renfermant de 1 à 8 atomes de carbone, m représentant le nombre 0, 1 ou 2 et quand m est 2, les substituants U peuvent être identiques ou différents, et les deux radicaux choisis parmi les radicaux R₁, R₂et R₃ qui ne représentent pas un radical : 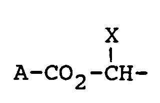 sont identiques ou différents et représentent ou un atome d'hydrogène ou un atome d'halogène ou un radical alkyle, alkényle ou alkynyle renfermant jusqu'à 8 atomes de carbone éventuellement substitué par un ou plusieurs atomes d'halogène ou un radical hydroxyle, O-alkyle, O-alkényle ou O-alkynyle, CO₂-alkyle, CO₂-alkényle, CO₂-alkynyle, S(O)ₙ-alkyle, S(O)ₙ-alkényle ou S(O)ₙ-alkynyle, n représentant le nombre 0, 1 ou 2, renfermant jusqu'à 8 atomes de carbone, éventuellement substitué par un ou plusieurs atomes d'halogène ou un radical aryle O-aryle ou thioaryle renfermant jusqu'à 14 atomes de carbone éventuellement substitué par un ou plusieurs radicaux choisis dans le groupe constitué par les atomes d'halogène, les radicaux alkyle, alkényle ou alkynyle renfermant jusqu'à 8 atomes de carbone éventuellement substitué par un ou plusieurs atomes d'halogène et les radicaux hydroxy libres estérifiés ou éthérifiés ou un radical hétéroaryle ou hétéroaryloxy ou un radical C≡N, NH₂ ou NO₂.

Lorsque X représente un radical alkyle, il s'agit de préférence du radical méthyle ou éthyle.

Lorsque X représente un radical alkynyle, il s'agit de préférence du radical éthynyle.

Lorsque T₁, T₂ ou Z₃ représentent un atome d'halogène, il s'agit de préférence d'un atome de fluor, de chlore ou de brome.

Lorsque T₁ ou T₂ représente un radical alkyle ou alkyloxy, il s'agit de préférence du radical méthyle, éthyle, propyle, méthoxy, éthoxy ou propoxy.

a, b, c et d représentent de préférence un atome de chlore ou de brome.

Lorsque D représente un atome d'halogène, il s'agit de préférence d'un atome de fluor, de chlore ou de brome.

Lorsque J représente un radical alkyle substitué par un ou plusieurs groupements fonctionnels, on entend de préférence par alkyle un radical renfermant de 1 à 8 atomes de carbone comme, par exemple, le radical méthyle, éthyle, propyle, isopropyle, butyle, isobutyle ou tert-butyle et par groupement fonctionnel l'un de ceux cités dans la demande européenne publiée sous le n° 50534.

J peut également représenter un radical alkyle susbtitué par un radical aryle, notamment radical phényle éventuellement substitué.

Lorsque J représente un radical alkyle substitué par un ou plusieurs groupements fonctionnels, on peut citer comme valeurs préférées de J, les radicaux :
-(CH₂)_{n₁}-C(Hal)₃ dans lequel n₁ est un entier de 1 à 8 et Hal un atome d'halogène, par exemple le radical -CH₂-CCl₃, -CH₂-CF₃, -CH₂-CH₂-CCl₃ ou CH₂-CH₂-CF₃ ;
-(CH₂)_{n₂}-CH(Hal)₂ dans lequel Hal est défini comme ci-dessus et n₂ est un nombre de 0 à 8, par exemple le radical -CH₂-CHCl₂, -CH₂-CHF₂ ou -CHF₂ ;
-(CH₂)_{n₁}-CH₂(Hal) dans lequel n₁ et Hal sont définis comme ci-dessus par exemple le radical -CH₂-CH₂Cl ou -CH₂-CH₂F,
-C(CHal₃)₃ dans lequel Hal est défini comme ci-dessus, par exemple le radical -C(CF₃)₃ ou -C(CF₃)₂-CCl₃,
-C(CF₃)₂-CH₃, -C(CH₃)₂-CF₃ ou -C(CH₃)(CF₃)-CH₂-CH₃,
-CH(CF₃)-CH₃ ou -CH(CF₃)₂, -C(CH₃)₂-CN, -CH(CH₃)-CN ou
-(CH₂)ₙ-CN
dans lequel n est défini comme précédemment, -CH(CN)-C(Hal)₃ dans lequel Hal est défini comme précédemment,
par exemple le radical : -CH(CN)-CCl₃
-(CH₂)_{n₁}-ORₐ, dans lequel n₁ est défini comme précédemment et Rₐ représente un atome d'hydrogène ou un radical alkyle linéaire ou ramifié, comportant de 1 à 8 atomes de carbone,
par exemple le radical -CH₂-OCH₃, -CH₂-CH₂-O-CH₃,
-CH₂-CH₂-O-CH₂-CH₃ ou -CH₂-CH₂-OH ; 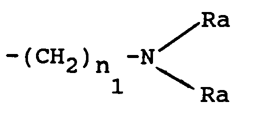
dans lequel n₁ et Rₐ sont définis comme précédemment et les deux radicaux Rₐ peuvent être différents entre eux, par exemple le radical :
-CH₂-CH₂-NH-CH₃, -CH₂-CH₂-N(CH₃)₂ ou
-CH₂-CH₂-N(CH₃)-CH₂-CH₃ ; 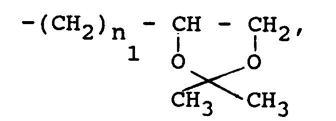
dans lequel n₁ est défini comme précédemment par exemple le radical : 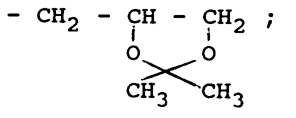
-(CH₂)_{n₁}-CH(OH)-CH₂-OH
dans lequel n₁ est défini comme précédemment par exemple le radical
-CH₂-CH(OH)-CH₂-OH ; -(CH₂)_{n₁}-O-THP
dans lequel n₁ est défini comme précédemment et THP représente le radical 2-tétrahydropyrannyle, par exemple le radical :
-CH₂-O-THP ou -CH₂-CH₂-O-THP ; 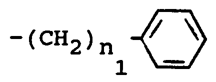
dans lequel n₁ est défini comme précédemment, par exemple le radical benzyle ou phénéthyle ; 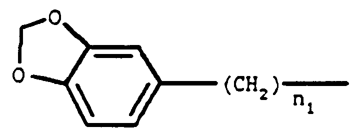
dans lequel n₁ est défini comme précédemment par exemple le radical :

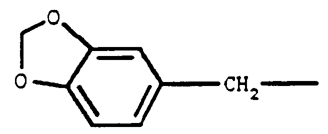

Lorsque J représente un radical aryle éventuellement substitué, il s'agit de préférence du radical phényle éventuellement substitué.

Lorsque J représente un radical hétérocyclique, il s'agit de préférence des radicaux pyridyle, furyle, thiényle, oxazolyle ou thiazolyle.

Dans la définition des substituants R₁, R₂ et R₃, lorsque ceux-ci ne représentent pas le radical : 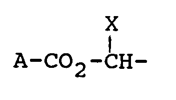
- halogène représente de préférence un atome de fluor, de chlore ou de brome,
- alkyle, alkényle et alkynyle représentent de préférence un radical méthyle, éthyle, isopropyle, n-butyle, isobutyle, tert-butyle, vinyle, allyle, éthynyle ou propynyle,
- radical alkyle substitué par un atome d'halogène, représente de préférence un radical CF₃, CHF₂, CHCl₂, CH₂Br, CH₂F ou encore 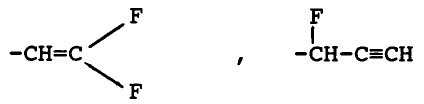
- aryle, représente de préférence un radical phényle éventuellement substitué par un ou plusieurs atomes d'halogène, par exemple le radical pentafluorophényle ou un radical phényle substitué par un ou plusieurs groupements CF₃ ou OCF₃,
- hétéroaryle représente paar exemple un radical thiényle, furyle, pyridyle, thiazolyle, tétrazolyle.

Parmi les composés préférés de l'invention, on peut citer
- les composés de formule (I) dans lesquels l'un des radicaux R₁, R₂ et R₃ représente un atome d'hydrogène,
- les composés de formule (I) dans lesquels celui des radicaux R₁, R₂ ou R₃ qui représente un radical : 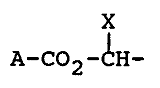 est situé en position 4,
- les composés de formule (I) dans lesquels l'un des radicaux R₁, R₂ ou R₃ représente un atome d'hydrogène en position 5,
- les composés de formule (I) dans lesquels l'un des radicaux R₁, R₂ ou R₃ représente un radical alkyle renfermant jusqu'à 4 atomes de carbone éventuellement substitué par un ou plusieurs atomes d'halogène,
- les composés de formule (I) dans lesquels l'un des radicaux R₁, R₂ ou R₃ représente un radical O-alkyle ou S-alkyle éventuellement substitué par un ou plusieurs atomes d'halogène,
- les composés de formule (I) dans lesquels le radical alkyle, O-alkyle ou S-alkyle est substitué par un ou plusieurs atomes de fluor,
- les composés de formule (I) dans lesquels l'un des radicaux R₁, R₂ ou R₃ représente un radical CF₃,
- les composés de formule (I) dans lesquels l'un des radicaux R₁, R₂ ou R₃ représente un radical OCHF₂,
- les composés de formule (I) dans lesquels X représente un radical éthynyle, ou dans lesquels X représente un atome d'hydrogène,
- les composés de formule (I) dans lesquels l'un des radicaux R₁, R₂ ou R₃ représente un radical :

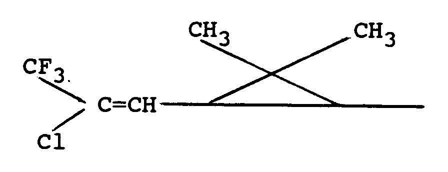

L'invention a plus particulièrement pour objet les composés de formule (I) dont la préparation est donnée ci-après dans la partie expérimentale et notamment le composé de l'exemple 1.

L'invention a également pour objet un procédé de préparation caractérisé en ce que l'on soumet un acide de formule (II) :

ACO₂H (II)

A étant défini comme précédemment, ou un dérivé fonctionnel de cet acide, à l'action d'un alcool de formule (III) : 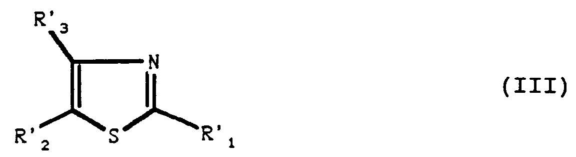
dans lequel l'un des radicaux R'₁, R'₂ ou R'₃ représente un radical : 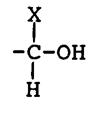
X conservant sa signification précécente et les deux autres radicaux ont la même signification que les radicaux R₁, R₂ et R₃ à l'exception de : 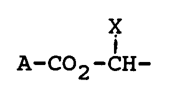
ou d'un dérivé fonctionnel de cet alcool de formule (III) pour obtenir le composé de formule (I) correspondant.

Le dérivé fonctionnel d'acide utilisé est de préférence un chlorure d'acide.

Lorsque l'on fait réagir l'acide de formule (II) sur l'alcool, on opère de préférence en présence de dicyclohexylcarbodiimide.

Les acides de formule (II) sont des produits connus, utilisés dans la synthèse des composés pyréthrinoïdes.

Les alcools de formule (III) sont des produits connus d'une façon générale qui peuvent être préparés par exemple selon les procédés décrits dans la demande de brevet européen 402.246 ou dans les brevets français 2 647 787, 2 289 189, 2 500 451 et 2 500 452.

Certains alcools de formule (III) dont la préparation est donnée ci-après sont des produits nouveaux et sont en eux-mêmes un objet de la présente invention.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères, comme Diabrotica, les taupins et les vers blancs, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait que les produits de formule (I) correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce de Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment les produits de formule (I) de l'exemple 1.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides et nématicides renfermant comme principe actif au moins un des produits de formule (I) définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2,2-1]5-heptène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention sans toutefois la limiter.

### EXEMPLE 1 : [1R-[1alpha, 3alpha(Z)]] 3-(2-chloro 3,3,3-trifluoro 1-propényl) 2,2-diméthyl cyclopropanecarboxylate de 1-[2-(trifluorométhyl) 4-thiazolyl] 2-propynyle isomère A et isomère B correspondants

On ajoute à 0°C, 0,41 g de dicyclohexylcarbodiimide et 5 cm³ de chlorure de méthylène dans une solution renfermant 0,48 g d'acide [1R-[1alpha, 3alpha(Z)]]-3-(2-chloro 3,3,3-trifluoro 1-propényl) 2,2-diméthyl cyclopropanecarboxylique, 0,41 g de alpha-éthynyl 2-(trifluorométhyl) 4-thiazoleméthanol préparé comme décrit ci-après, 20 cm³ de chlorure de méthylène et 20 mg de diméthylaminopyridine (DMAP).

On maintient le mélange réactionnel sous agitation à 0°C pendant 30 minutes et laisse revenir à 20 ≃ 25°C. On filtre et amène à sec le produit obtenu. On chromatographie sur silice en éluant avec le mélange hexane-acétate d'éthyle (9-1). On obtient 0,6 g de produit recherché. Rf = 0,12.
RMN :
- H des méthyles géminés: 1,28 ; 1,30 et 1,32 ppm
- H en 1 et 3 du cyclopropane: 2,06 (d,J = 8,5 Hz) et 2,22 ppm (m)
- H du carbone portant l'éthynyle: 6,56 ppm et 6,61 ppm (d,J = 1,5 Hz)
- H de l'éthynyle: 2,71 ppm (d,J = 1,5 Hz)
- H éthylényle (ΔZ): 6,88 ppm (d,J = 9,5 Hz)
- H en alpha du soufre: 7,79 ppm et 7,80 ppm

Les isomères R et S correspondants ont été séparés par chromatographie sur silice en éluant avec le mélange heptane-t-butylméthyléther (95-5). On obtient un produit A Rf = 0,16 et un produit B. Rf = 0,12.

### PREPARATION DE L'EXEMPLE 1 : alpha-éthynyl 2-(trifluorométhyl) 4-thiazoleméthanol

On ajoute 11 cm³ d'une solution molaire de bromure d'éthynyl magnésium dans une solution de tétrahydrofuranne (THF) renfermant 2 g de 2-(trifluorométhyl) 4-thiazolecarboxaldéhyde. On maintient le mélange réactionnel sous agitation pendant 30 minutes à 20 ≃ 25°C. On verse sur une solution de chlorure d'ammonium. On extrait au chlorure de méthylène, sèche, filtre et amène à sec. On obtient 2,1 g de produit recherché.

En opérant comme à l'exemple 1, on a obtenu les composés suivants :

### EXEMPLE 2 : [1R-[1alpha, 3alpha(Z)]]-3-(3-méthoxy 3-oxo 1-propényl) 2,2-diméthyl cyclopropanecarboxylate de [2-(trifluorométhyl) 4-thiazolyl] méthyle

F = 117°C.

### EXEMPLE 3 : [1R-[1alpha, 3alpha(Z)]]-3-(2-chloro 3,3,3-trifluoro 1-propényl) 2,2-diméthyl cyclopropanecarboxylate de [2-(trifluorométhyl) 4-thiazolyl] méthyle

alpha_{D} = +28° (c = 0,35 % CHCl₃).

### EXEMPLE 4 : [1R-[1alpha, 3alpha(E)]]-3-[2-fluoro 3-(1,1-diméthyl éthoxy) 3-oxo 1-propényl] 2,2-diméthyl cyclopropanecarboxylate de [2-(trifluorométhyl) 4-thiazolyl] méthyle

alpha_{D} = +48° (c = 0,35 % CHCl₃).

### EXEMPLE 5 : [1R-[1alpha, 3alpha(Z)]] 2,2-diméthyl 3-[3-oxo 3-[2,2,2-trifluoro 1-(trifluorométhyl) éthoxy] 1-propényl] cyclopropanecarboxylate de [2-(trifluorométhyl) 4-thiazolyl] méthyle

alpha_{D} = +35° (c = 0,25 % CHCl₃).

### EXEMPLE 6 : [1R-[1alpha, 3alpha]]-3-(2,2-dichloro éthényl) 2,2-diméthyl cyclopropanecarboxylate de [2-(trifluorométhyl) 4-thiazolyl] méthyle

alpha_{D} = +8° (c = 0,2 % CHCl₃).

### EXEMPLE 7 : [1R-[1alpha, 3alpha]]-3-(2,2-dichloro éthényl) 2,2-diméthyl cyclopropanecarboxylate de [2-(pentafluorophényl) 4-thiazolyl] méthyle

alpha_{D} = -2,5° (c = 0,9 % CHCl₃).

### EXEMPLE 8 : [1R-[1alpha, 3alpha(Z)]]-3-(2-chloro 3,3,3-trifluoro 1-propényl) 2,2-diméthyl cyclopropanecarboxylate de [2-(pentafluorophényl) 1-thiazolyl] méthyle

alpha_{D} = +16° (c = 0,6 % CHCl₃).

### EXEMPLE 9 : [1R-[1alpha, 3alpha(E)]]-3-[2-fluoro 3-(1,1-diméthyl éthoxy) 3-oxo 1-propényl] 2,2-diméthyl carboxylate de [2-(pentafluorophényl) 1-thiazolyl] méthyle

F = 101°C.

### EXEMPLE 10 : [1R-[1alpha, 3alpha(Z)]]-3-(3-méthoxy 3-oxo 1-propényl) 2,2-diméthyl cyclopropanecarboxylate de [2-(2,3,4,5,6-pentafluorophényl) 4-thiazolyl] méthyle

alpha_{D} = 42,5° (c = 0,6 % CHCl₃).

### EXEMPLE 11 : [1R-[1alpha, 3alpha(Z)]] 2,2-diméthyl 3-[3-oxo 3-[2,2,2-trifluoro 1-(trifluorométhyl) éthoxy] 1-propényl] cyclopropanecarboxylate de [2-(pentafluorophényl) 4-thiazolyl] méthyle

Rf = 0,1 hexane-acétate d'éthyle (9-1).

### EXEMPLE 12 : [1R-[1alpha, 3alpha(Z)]]-3-(2-chloro 3,3,3-trifluoro 1-propényl) 2,2-diméthyl cyclopropanecarboxylate de [2-(difluorométhoxy) 4-thiazolyl] méthyle

Rf = 0,3 hexane-chlorure de méthylène (50-50).

### PREPARATION DE L'EXEMPLE 12 : 2-(difluorométhoxy) 4-thiazoleméthanol

L'alcool utilisé au départ de l'exemple 12 a été préparé comme décrit ci-dessous :

### STADE A : 2-(difluorométhoxy) 4-thiazolecarboxylate d'éthyle

On chauffe vers 80°C un mélange renfermant 1,2 g de 2-oxo 4-thiazolecarboxylate d'éthyle, 3 g de carbonate de potassium et 10 ml de diméthylformamide (DMF) anhydre en présence de chlorodifluorométhane. Une fois la réaction terminée, on verse le milieu réactionnel sur l'eau, extrait à l'acétate d'éthyle, lave à l'eau, sèche et amène à sec. On isole 3 g d'un produit que l'on purifie par chromatographie sur silice en éluant à l'aide d'un mélange hexane-acétate d'éthyle (80-20). On obtient ainsi 1,2 g de produit recherché. Rf = 0,3.

### STADE B : 2-(difluorométhoxy) 4-thiazoleméthanol

On introduit à -60°C, 7,2 ml d'une solution 1,5 molaire d'hydrure de diisobutylaluminium (DIBAL) dans le toluène dans un mélange renfermant 1,2 g du produit préparé au stade A et 35 ml de toluène anhydre. On laisse remonter la température à -30°C, maintient le mélange réactionnel à -30°C pendant 2 heures et verse sur une solution molaire de tartrate double de sodium et de potassium refroidie à 0°C. On agite 2 heures à température ambiante, extrait à l'acétate d'éthyle, lave à l'eau, sèche et amène à sec. On isole 2,4 g de produit que l'on purifie par chromatographie sur silice en éluant avec le mélange hexane-acétate d'éthyle (50-50). On obtient 840 mg de produit recherché.
Rf = 0,2 éluant hexane-acétate d'éthyle (50-50).

### EXEMPLE 13 : [1R-[1alpha, 3alpha(E)]]-3-(3-éthoxy 2-fluoro 3-oxo 1-propényl) 2,2-diméthyl cyclopropanecarboxylate de [2-(difluorométhoxy) 4-thiazolyl] méthyle

Rf = 0,25 hexane-chlorure de méthylène (20-80).

### EXEMPLE 14 : [1R-[1alpha, 3alpha(Z)]]-3-(2-chloro 3,3,3-trifluoro 1-propényl) 2,2-diméthyl cyclopropanecarboxylate de 1-[2-(difluorométhoxy) 4-thiazolyl] 2-propynyle

Rf = 0,3 hexane-chlorure de méthylène (50-50).

### PREPARATION DE L'EXEMPLE 14 : 2-(difluorométhoxy) alpha-éthynyl 4-thiazoleméthanol

L'alcool utilisé au départ de l'exemple 14 a été préparé comme décrit ci-après :
a) Préparation de l'aldéhyde
   A une suspension renfermant 3 g de chlorochromate de pyridinium et 50 cm³ de chlorure de méthylène, on ajoute 50 cm³ d'une solution de chlorure de méthylène renfermant 2,47 g de 2-(difluorométhoxy) 4-thiazoleméthanol. On maintient le mélange réactionnel pendant 1 h 30 à 25°C, filtre, évapore le chlorure de méthylène, purifie le produit obtenu sur silice en éluant avec un mélange hexane-acétate d'éthyle (50-50). On isole 2,6 g du produit recherché.
b) Stade d'éthynylation
   On ajoute à 0°C, 13 ml d'une solution 0,5 M dans le THF de bromure d'éthynylmagnésium, dans un mélange renfermant 6 mmoles de produit préparé au stade A et 6 ml de THF anhydre. On maintient le mélange réactionnel sous agitation à 0°C, verse le milieu réactionnel sur une solution aqueuse saturée en chlorure d'ammonium, extrait à l'acétate d'éthyle, lave, sèche sur sulfate de magnésium et amène à sec. On chromatographie le produit obtenu sur silice, en éluant avec un mélange hexane-acétate d'éthyle (70-30). On isole ainsi 720 mg de produit recherché. Rf = 0,25 (hexane-acétate d'éthyle 7-3).

### EXEMPLE 15 : [1R-[1alpha, 3alpha(Z)]]-3-(2-chloro 3,3,3-trifluoro 1-propényl) 2,2-diméthyl cyclopropanecarboxylate de 1-[2-(difluorométhoxy) 4-thiazolyl] éthyle

En opérant comme dans l'exemple 1, à partir de la solution préparée ci-après et de l'acide [1R-(1alpha, 3alpha(Z)]-3-(2-chloro 3,3,3-trifluoro 1-propényl) 2,2-diméthyl cyclopropanecarboxylique, on obtient le produit recherché. Rf = 0,3 (éluant : hexane-chlorure de méthylène (70-30)).

### PREPARATION DE L'EXEMPLE 15 : 1-[2-(difluorométhoxy) 4-thiazolyl] éthanol

On ajoute à 0 ± 5°C, une solution 3M de bromure de méthylmagnésium dans le THF, dans une solution renfermant 6 mmoles de l'aldéhyde préparé au stade a) de la préparation de l'exemple 14 dans 15 ml de THF. On maintient le mélange réactionnel sous agitation pendant 15 minutes à 0°C, verse sur une solution aqueuse à 10 % de chlorure d'ammonium, extrait à l'éther éthylique et sèche sur sulfate de magnésium. On isole 70 ml d'une solution renfermant l'alcool recherché que l'on utilise tel quel dans le stade suivant.

### EXEMPLE 16 : [1R-[1alpha, 3alpha(Z)]]-3-(2-chloro 3,3,3-trifluoro 1-propényl) 2,2-diméthyl cyclopropanecarboxylate de (4-thiazolyl) méthyle

Rf = 0,08 hexane-acétate d'éthyle (9-1).

### EXEMPLE 17 : [1R-[1alpha, 3alpha(Z)]]-3-(2-chloro 3,3,3-trifluoro 1-propényl) 2,2-diméthyl cyclopropanecarboxylate de [2-(1-méthyl éthyl) 4-thiazolyl] méthyle

alpha_{D} = + 19° (c = 1,3 % CHCl₃).

### EXEMPLE 18 : [1R-[1alpha, 3alpha(Z)]]-3-(2-chloro 3,3,3-trifluoro 1-propényl) 2,2-diméthyl cyclopropanecarboxylate de (2-fluoro 4-thiazolyl) méthyle

Rf = 0,25 hexane-chlorure de méthylène (50-50).

### PREPARATION DE L'EXEMPLE 18 : 2-fluoro 4-thiazoleméthanol

L'alcool utilisé au départ de l'exemple 18 a été préparé comme décrit ci-dessous :

### STADE A : 2-fluoro 4-thiazolecarboxylate d'éthyle

On chauffe à 140°C pendant 20 heures une suspension renfermant 2,1 g de 2-chloro 4-thiazolecarboxylate d'éthyle, 15 ml de diméthylsulfoxyde (DMSO) et 2 g de fluorure de potassium. On chromatographie le produit obtenu en éluant avec le mélange hexane-acétate d'éthyle (80-20). On isole 0,82 g du produit recherché. F = 74°C.

### STADE B : 2-fluoro 4-thiazoleméthanol

On refroidit à -60°C, une solution renfermant 600 mg de produit préparé au stade A et 20 ml de THF. On introduit à -60°C, 4,5 cm³ de DIBAL 1,5 molaire dans le toluène. On laisse la température remonter vers -10°/-20°C. On ajoute 34 mg de borohydrure de sodium, agite pendant 30 minutes à 0°/-5°C, verse sur une solution molaire de tartrate double de sodium et de potassium. On agite pendant 1 heure à la température ambiante, extrait à l'acétate d'éthyle, lave, sèche sur sulfate de magnésium et amène à sec. On chromatographie le produit obtenu en éluant avec le mélange hexane-acétate d'éthyle (50-50). On obtient 450 mg de produit recherché.
Rf = 0,25.

### EXEMPLE 19 : [1R-[1alpha, 3alpha(Z)]]-3-(2-chloro 3,3,3-trifluoro 1-propényl) 2,2-diméthyl cyclopropanecarboxylate de (2-phénoxy 4-thiazolyl) méthyle

alpha_{D} = + 16,5° (c = 1,1 % CHCl₃).

### EXEMPLE 20 : [1R-[1alpha, 3alpha(Z)]]-3-(2-chloro 3,3,3-trifluoro 1-propényl) 2,2-diméthyl cyclopropanecarboxylate de [2-[4-(trifluorométhoxy) phényl] 4-thiazolyl] méthyle

alpha_{D} = + 12,5° (c = 1,15 % CHCl₃).

### EXEMPLE 21 : [1R-[1alpha, 3alpha(Z)]]-3-(2-chloro 3,3,3-trifluoro 1-propényl) 2,2-diméthyl cyclopropanecarboxylate de [2-[3-(trifluorométhoxy) phényl] 4-thiazolyl] méthyle

alpha_{D} = + 16,5° (c = 1 % CHCl₃).

### EXEMPLE 22 : [1R-[1alpha, 3alpha(Z)]]-3-(2-chloro 3,3,3-trifluoro 1-propényl) 2,2-diméthyl cyclopropanecarboxylate de [2-[3-(trifluorométhyl) phényl] 4-thiazolyl] méthyle

alpha_{D} = + 14,5° (c = 1,15 % CHCl₃).

### EXEMPLE 23 : [1R-[1alpha, 3alpha(Z)]]-3-(2-chloro 3,3,3-trifluoro 1-propényl) 2,2-diméthyl cyclopropanecarboxylate de [2-[4-(trifluorométhyl) phényl] 4-thiazolyl] méthyle

alpha_{D} = + 14° (c = 1,2 % CHCl₃).

### EXEMPLE 24 : [1R-[1alpha, 3alpha(Z)]]-3-(2-chloro 3,3,3-trifluoro 1-propényl) 2,2-diméthyl cyclopropanecarboxylate de [2-[2-(trifluorométhyl) phényl] 4-thiazolyl] méthyle

alpha_{D} = + 19,5° (c = 0,85 % CHCl₃).

### EXEMPLE 25 : [1R-[1alpha, 3alpha(Z)]]-3-(2-chloro 3,3,3-trifluoro 1-propényl) 2,2-diméthyl cyclopropanecarboxylate de [2-(2-éthyl 4-thiazolyl) 4-thiazolyl] méthyle

alpha_{D} = + 11,5° (c = 1 % CHCl₃).

### EXEMPLE 26 : [1R-[1alpha, 3alpha(Z)]]-3-(2-chloro 3,3,3-trifluoro 1-propényl) 2,2-diméthyl cyclopropanecarboxylate de (2-chloro 4-thiazolyl) méthyle

alpha_{D} = + 24,5° (c = 1,05 % CHCl₃).

### EXEMPLE 27 : [1R-[1alpha, 3alpha(Z)]]-3-(2-chloro 3,3,3-trifluoro 1-propényl) 2,2-diméthyl cyclopropanecarboxylate de [2-(2-thiényl) 4-thiazolyl] méthyle

alpha_{D} = + 14,5° (c = 1 % CHCl₃).

### EXEMPLE 28 : [1R-[1alpha, 3alpha(Z)]]-3-(2-chloro 3,3,3-trifluoro 1-propényl) 2,2-diméthyl cyclopropanecarboxylate de (2-méthylthio 4-thiazolyl) méthyle

alpha_{D} = + 20° (c = 1,05 % CHCl₃).

### EXEMPLE 29 : [1R-[1alpha, 3alpha(Z)]]-3-(2-chloro 3,3,3-trifluoro 1-propényl) 2,2-diméthyl cyclopropanecarboxylate de (2-méthyl 4-thiazolyl) méthyle

Rf = 0,14 hexane-acétate d'éthyle (9-1).

### EXEMPLE 30 : [1R-[1alpha, 3alpha(Z)]]-3-(2-chloro 3,3,3-trifluoro 1-propényl) 2,2-diméthyl cyclopropanecarboxylate de (2-chloro 5-thiazolyl) méthyle

alpha_{D} = + 59° (c = 1,1 % CHCl₃).

### EXEMPLE 31 : 1R-[1alpha, 3alpha(Z)]-3-(2-chloro 3,3,3-trifluoro 1-propényl) 2,2-diméthyl cyclopropanecarboxylate de (5-thiazolyl) méthyle

alpha_{D} = + 45° (c = 1,1 % CHCl₃).

### EXEMPLE 32 : [1R-[1alpha, 3alpha(Z)]]-3-(2-chloro 3,3,3-trifluoro 1-propényl) 2,2-diméthyl cyclopropanecarboxylate de (2-phénoxy 5-thiazolyl) méthyle

Rf = + 14°C (c = 1 % CHCl₃).

### EXEMPLE 33 : [1R-[1alpha, 3alpha(E)]]-3-(3-éthoxy 2-fluoro 3-oxo 1-propényl) 2,2-diméthyl cyclopropanecarboxylate de (2-phénoxy 5-thiazolyl) méthyle

alpha_{D} = + 29° (c = 1 % CHCl₃).

### EXEMPLE 34 : [1R-[1alpha, 3alpha(E)]]-3-(3-éthoxy 2-fluoro 3-oxo 1-propényl) 2,2-diméthyl cyclopropanecarboxylate de (4-thiazolyl) méthyle

Rf = 0,13 hexane-acétate d'éthyle (8-2).

### EXEMPLE 35 : [1R-[1alpha, 3alpha(E)]]-3-(3-éthoxy 2-fluoro 3-oxo 1-propényl) 2,2-diméthyl cyclopropanecarboxylate de [2-(1-méthyl éthyl) 4-thiazolyl] méthyle

Rf = 0,29 hexane-acétate d'éthyle (8-2).

### EXEMPLE 36 : [1R-[1alpha, 3alpha(Z)]]-3-(2-chloro 3,3,3-trifluoro 1-propényl) 2,2-diméthyl cyclopropanecarboxylate de 1-(2-méthyl 4-thiazolyl) 2-propynyle

Rf = 0,21 heptane-acétate d'éthyle (8-2).

### PREPARATION DE L'EXEMPLE 36 : alpha-éthynyl 2-méthyl 4-thiazoleméthanol

En opérant comme à la préparation de l'exemple 1, à partir du 2-méthyl 4-thiazolecarboxaldéhyde, on a obtenu le produit recherché. F = 94°C.

### EXEMPLE 37 : [1R-[1alpha, 3alpha(E)]]-3-(3-éthoxy 2-fluoro 3-oxo 1-propényl) 2,2-diméthyl cyclopropanecarboxylate de 1-[2-(trifluorométhyl) 4-thiazolyl] éthyle

Rf = 0,13 hexane-acétate d'éthyle (8-2).

### PREPARATION DE L'EXEMPLE 37 : 1-[2-(trifluorométhyl) 4-thiazolyl] éthanol

En opérant comme à la préparation de l'exemple 1 à partir du 2-(trifluorométhyl) 4-thiazolecarboxaldéhyde et du bromure de méthyl magnésium, on a obtenu le produit recherché.
Rf = 0,17 hexane-acétate d'éthyle (7-3).

### EXEMPLE 38 : [1R-[1alpha, 3alpha(Z)]]-3-(2-chloro 3,3,3-trifluoro 1-propényl) 2,2-diméthyl cyclopropanecarboxylate de 1-(2-bromo 4-thiazolyl) 2-propynyle

F = 76°C.

### PREPARATION DE L'EXEMPLE 38 : 2-bromo alpha-éthynyl 4-thiazoleméthanol

En opérant comme à la préparation de l'exemple 1 à partir du 2-bromo 4-thiazolecarboxaldéhyde et du bromure d'éthynyl magnésium, on a obtenu le produit recherché.
Rf = 0,17 hexane-acétate d'éthyle (7-3).

### EXEMPLE 39 : [1R-[1alpha, 3alpha(E)]]-3-(3-éthoxy 2-fluoro 3-oxo 1-propényl) 2,2-diméthyl cyclopropanecarboxylate de (2-bromo 4-thiazolyl) méthyle

Rf = 0,11 heptane-acétate d'éthyle (9-1).

### PREPARATION DE L'EXEMPLE 39 : 2-bromo 4-thiazoleméthanol

### STADE A : 2-bromo 4-thiazolecarboxylate d'éthyle

On chauffe 4 heures au reflux un mélange contenant 15 ml de dichloroéthane dans un mélange renfermant 2,87 g de d'oxybromure de phosphore et 1,73 g de 2-oxo 4-thiazolecarboxylate d'éthyle. On maintient le mélange réactionnel au reflux pendant 4 heures et le verse sur un mélange d'eau et de glace. On extrait au chlorure de méthylène, sèche sur sulfate de magnésium, filtre et amène à sec. On chromatographie le produit obtenu, sur silice en éluant avec le mélange hexane-acétate d'éthyle (7-3). On obtient 1,2 g de produit recherché.
F = 69°C.

### STADE B : 2-bromo 4-thiazoleméthanol et 2-bromo 4-thiazolecarboxaldéhyde

On ajoute à -60°C, 141 ml de DIBAL 1,2 molaire dans l'hexane dans une solution renfermant 20 g de produit préparé au stade A et 200 ml de tétrahydrofuranne. On maintient le mélange réactionnel sous agitation pendant 1 heure. On laisse la température remonter à -20°C. On verse le mélange réactionnel sur une solution molaire de tartrate double de sodium et de potassium. On extrait à l'acétate d'éthyle, sèche, filtre et amène à sec. On chromatographie le produit obtenu sur silice en éluant avec le mélange hexane-acétate d'éthyle (7-3). On obtient ainsi 14,9 g de 2-bromo 4-thiazoleméthanol (Rf = 0,06) et 1 g de 2-bromo 4-thiazolecarboxaldéhyde.
Rf = 0,29. F = 124°C.

### EXEMPLE 40 : [1R-[1alpha, 3alpha(E)]]-3-(3-éthoxy 2-fluoro 3-oxo 1-propényl) 2,2-diméthyl cyclopropanecarboxylate de [2-[4-(trifluorométhoxy) phényl] 4-thiazolyl] méthyle Rf = 0,34 hexane-acétate d'éthyle (8-2).

### EXEMPLE 41 : [1R-[1alpha, 3alpha(E)]]-3-(3-éthoxy 2-fluoro 3-oxo 1-propényl) 2,2-diméthyl cyclopropanecarboxylate de (2-chloro 4-thiazolyl) méthyle

Rf = 0,30 hexane-acétate d'éthyle (8-2).

### EXEMPLE 42 : [1R-[1alpha, 3alpha(E)]]-3-(3-éthoxy 2-fluoro 3-oxo 1-propényl) 2,2-diméthyl cyclopropanecarboxylate de (2-méthylthio 4-thiazolyl) méthyle

F = 76°C.

### EXEMPLE 43 : [1R-[1alpha, 3alpha(Z)]]-3-(2-chloro 3,3,3-trifluoro 1-propényl) 2,2-diméthyl cyclopropanecarboxylate de 1-[2-(trifluorométhyl) 4-thiazolyl] éthyle

Rf = 0,09 heptane-acétate d'éthyle (9-1).

### EXEMPLE 44 : [1R-[1alpha, 3alpha(Z)]]-3-(2-chloro 3,3,3-trifluoro 1-propényl) 2,2-diméthyl cyclopropanecarboxylate de (2-bromo 4-thiazolyl) méthyle

Rf = 0,17 heptane-acétate d'éthyle (9-1).

On utilise l'alcool obtenu à la préparation de l'exemple 39.

### EXEMPLE 45 : [1R-[1alpha, 3alpha(Z)]]-3-(2-chloro 3,3,3-trifluoro 1-propényl) 2,2-diméthyl cyclopropanecarboxylate de cyano [2-(trifluorométhyl) 4-thiazolyl] méthyle

F < 50°C.
Rf = 0,32 (hexane-acétate d'éthyle 8-2).

### EXEMPLE 46 : [1R-[1alpha, 3alpha(Z)]]-3-(2-chloro 3,3,3-trifluoro 1-propényl) 2,2-diméthyl cyclopropanecarboxylate de [2-(1-fluoro 1-propynyl) 4-thiazolyl] méthyle

Rf = 0,11 hexane-acétate d'éthyle (8-2).

### PREPARATION DE L'EXEMPLE 46 : 2-(1-fluoro 2-propynyl) 4-thiazoleméthanol

### STADE A : 2-(bromométhyl) 4-[[(tétrahydro 2H-pyran-2-yl) oxy] méthyl] thiazole

Le produit a été préparé par action du dihydropyranne et de l'alcool correspondant au produit recherché.
Rf = 0,46 hexane-acétate d'éthyle (1-1).

### STADE B : 4-[[(tétrahydro 2H-pyran-2-yl) oxy] méthyl] 2-thiazolecarboxaldéhyde

On agite à 20°C, pendant 1 heure un mélange renfermant 5 g du produit préparé au stade A, 50 cm³ de chlorure de méthylène et 9,5 g de N-oxyde N-méthyl morpholine monohydraté. On amène à sec le produit obtenu et chromatographie le résidu sur silice en éluant avec le mélange cyclohexane-acétate d'éthyle (7-3). On obtient 2,8 g de produit recherché. Rf = 0,27.

### STADE C : alpha-éthynyl 4-[[(tétrahydro 2H-pyran-2-yl) oxy] méthyl] 2-thiazoleméthanol

Le produit a été préparé à partir du produit préparé au stade B par action du bromure d'éthynylmagnésium en suivant le mode opératoire de la préparation de l'exemple 1.
Rf = 0,11 (hexane-acétate d'éthyle (7-3)).

### STADE D : 2-(1-fluoro 2-propynyl) 4-thiazoleméthanol

On ajoute à -60°C environ, 0,53 ml de diéthylaminosulfure trifluorure (DAST) dans une solution renfermant 1 g du produit préparé au stade C et 5 cm³ de chlorure de méthylène. On maintient le mélange réactionnel sous agitation pendant 2 heures à -55°C. On le verse sur une solution de bicarbonate de sodium. On extrait au chlorure de méthylène, sèche sur sulfate de magnésium, filtre et amène à sec. On reprend le résidu obtenu dans 5 ml de méthanol et ajoute 20 mg d'acide paratoluènesulfonique. On maintient le mélange réactionnel sous agitation pendant 1 heure à 20°C. On amène à sec. On chromatographie le produit obtenu sur silice en éluant avec le mélange hexane-acétate d'éthyle (7-3). On obtient 0,16 g de produit recherché. Rf = 0,10.

### EXEMPLE 47 : 1R[1alpha,3alpha(Z)] 2,2-diméthyl 3-(3,3,3-trifluoro 2-chloropropényl) cyclopropane carboxylate de 1-(méthylthio 4-thiazolyl) 2-propynyle.

Rf = 0,16 (heptane-AcOEt 8-2).

### PREPARATION DE L'EXEMPLE 47 : (2-méthylthio) alpha-éthynyl 4-thiazol méthanol.

On fait réagir du thiométhoxyde de sodium sur le 2-chloro 4-thiazol carboxylate d'éthyle puis du borohydrure de sodium en présence de méthanol pour obtenir le 2-méthylthio 4-thiazolyl méthanol que l'on traite par l'oxyde de manganèse pour obtenir l'aldéhyde puis par le bromure d'éthynyl magnésium comme indiqué à la préparation 14 pour obtenir l'alcool attendu.

### EXEMPLE 48 : [1R-[1alpha,3alpha(E)]] 3-(3-éthoxy 2-fluoro 3-oxo 1-propényl) 2,2-diméthyl cyclopropane carboxylate de 1-(2-méthylthio 4-thiazolyl) 2-propynyle.

Rf = 0,13 (heptane-AcOEt 8-2).

### EXEMPLE 49 : 1R[1alpha,3alpha(Z)] 2,2-diméthyl 3-(3,3,3-trifluoro 2-chloropropényl) cyclopropane carboxylate de 1-(2-fluoro 4-thiazolyl) 2-propynyle.

Rf = 0,2 (CH₂Cl₂-hexane 5-5).

### PREPARATION DE L'EXEMPLE 49 : 2-fluoro alpha-éthynyl 4-thiazol méthanol.

En opérant comme à la préparation 14 à partir de l'alcool obtenu à la préparation 18, on obtient l'alcool attendu.

### EXEMPLE 50 : 1R[1alpha,3alpha(Z)] 2,2-diméthyl 3-(3,3,3-trifluoro 2-chloropropényl) cyclopropane carboxylate de 1-(2-chloro 4-thiazolyl) 2-propynyle.

Rf = 0,2 (CH₂Cl₂-heptane 5-5).

### PREPARATION DE L'EXEMPLE 50 : 2-chloro alpha-éthynyl 4-thiazol méthanol.

On traite le 2-chloro 4-thiazol carboxylate d'éthyle par du borohydrure de sodium en présence de méthanol pour obtenir l'aldéhyde correspondant puis par le bromure d'éthynyl magnésium comme indiqué à la préparation 14 pour obtenir l'alcool attendu.

### EXEMPLE 51 : 1R[1alpha,3alpha(Z)] 2,2-diméthyl 3-(3,3,3-trifluoro 2-chloropropényl) cyclopropane carboxylate de 1-(2-chloro 4-thiazolyl) éthyle.

Rf = 0,2 (CH₂Cl₂-heptane 5-5).

### EXEMPLE 52 : 1R[1alpha,3alpha(Z)] 2,2-diméthyl 3-(3,3,3-trifluoro 2-chloropropényl) cyclopropane carboxylate de 1-(2-éthynyl 4-thiazolyl) méthyle.

Rf = 0,2 (CH₂Cl₂-heptane 8-2).

### PREPARATION DE L'EXEMPLE 52 : 2-éthynyl 4-thiazol méthanol.

On traite le 2-bromo 4-thiazolyl méthanol de la préparation 39 par du triméthylacétylène en présence d'iodure de cuivre, de chlorure de bis (triphénylphosphine) palladium et de triéthylamine puis verse dans une solution aqueuse de chlorure d'ammonium pour obtenir l'alcool attendu.

### EXEMPLE 53 : 1R[Aalpha,3alpha(Z)] 2,2-diméthyl 3-(3,3,3-trifluoro 2-chloropropényl) cyclopropane carboxylate de 1-(2-éthynyl 4-thiazolyl) 2-propynyle.

Rf ≈ 0,2 (heptane-CH₂Cl₂ 4-6).

### PREPARATION DE l'EXEMPLE 53 : 2-éthynyl alpha-éthynyl 4-thiazol méthanol.

En opérant comme à la préparation 14 à partir de l'alcool obtenu à la préparation 52, on obtient l'alcool attendu.

### EXEMPLE 54 : 1R[Aalpha,3alpha(Z)] 2,2-diméthyl 3-(3,3,3-trifluoro 2-chloropropényl) cyclopropane carboxylate de 1-(2-chloro 4-thiazolyl) R et S cyano méthyle.

Rf = 0,25 (CH₂Cl₂-heptane 7-3).

### EXEMPLE 55 : [1R-[1alpha,3alpha(E)]] 3-(3-éthoxy 2-fluoro 3-oxo 1-propényl) 2,2-diméthyl cyclopropane carboxylate de 1-(2-bromo 4-thiazolyl) 2-propynyle.

Rf = 0,19 (heptane-AcOEt 9-1).

### EXEMPLE 56 : 1R[1alpha,3alpha(Z)] 2,2-diméthyl 3-(3,3,3-trifluoro 2-chloropropényl) cyclopropane carboxylate de [2-(pentafluoroéthyl) 4-thiazolyl) méthyle.

Rf = 0,14 (heptane-CH₂Cl₂ 7-3).

### PREPARATION DE L'EXEMPLE 56 : 2-pentafluoroéthyl 4-thiazol méthanol et 2 pentafluoroéthyl 4-thiazol carboxaldéhyde.

On fait agir le bromopyruvate d'éthyle sur le pentafluoropropane thioamide en milieu éthanolique pour obtenir le 2-pentafluoroéthyl 4-thiazol carboxylate d'éthyle puis opère comme au stade B de la préparation 39 pour obtenir les produits attendus.

### EXEMPLE 57 : 1R[1alpha,3alpha(Z)] 2,2-diméthyl 3-(3,3,3-trifluoro 2-chloropropényl) cyclopropane carboxylate de 1-[2-(pentafluoroéthyl) 4-thiazolyl) 2-propynyle.

Rf = 0,12 (heptane-AcOEt 9-1).

### PREPARATION DE L'EXEMPLE 57 : 2-pentafluoroéthyl alpha-éthynyl 4-thiazol méthanol.

En opérant comme à la préparation 1 à partir du produit obtenu à la préparation 56, on obtient l'alcool attendu.

### EXEMPLE 58 : 1R[1alpha,3alpha(E)] 3-(2-fluoro 3-méthoxy 3-oxo 1-propényl) 2,2-diméthyl cyclopropane carboxylate de 1-(2-difluorométhoxy) 4-thiazolyl) 2-propynyle.

### PREPARATION DE L'EXEMPLE 58 : 2-(difluorométhyl) alpha-éthynyl 4-thiazol méthanol.

En opérant comme à la préparation 1 à partir du 2-(difluorométhyl) 4-thiazol carboxaldéhyde, on obtient l'alcool attendu.

### EXEMPLE 59 : 1R[1alpha,3alpha(Z)] 2,2-diméthyl 3-(3,3,3-trifluoro 2-chloropropényl) cyclopropane carboxylate de (2-difluorométhyl) 4-thiazolyl) méthyle.

Rf = 0,12 (heptane-AcOEt 95-5).

### EXEMPLE 60 : 1R[1alpha,3alpha(E)] 3-(2-fluoro 3-méthoxy 3-oxo 1-propényl) 2,2-diméthyl cyclopropane carboxylate de (2-difluorométhyl) 4-thiazolyl) méthyle.

Rf = 0,10 (heptane-AcOEt 95-5).

### EXEMPLE 61 : 1R[1alpha,3alpha(E)] 3-(2-fluoro 3-méthoxy 3-oxo 1-propényl) 2,2-diméthyl cyclopropane carboxylate de 1-(2-difluorométhyl) 4-thiazolyl) 2-propynyle.

Rf = 0,11 (heptane-CH₂Cl₂ 4-6).

### EXEMPLE 62 : 1R[1alpha,3alpha(Z)] 2,2-diméthyl 3-(3,3,3-trifluoro 2-chloropropényl) cyclopropane carboxylate de 1-(2-difluorométhyl) 4-thiazolyl) 2-propynyle.

Rf = 0,12 (heptane-CH₂Cl₂ 5-5).

### EXEMPLE 63 : 1R[1alpha,3alpha(Z)] 2,2-diméthyl 3-(3,3,3-trifluoro 2-chloropropényl) cyclopropane carboxylate de (3-difluorométhyl) 2-oxo 5-thiazolyl) méthyle.

Rf = 0,19 (heptane-CH₂Cl₂ 1-1).

### PREPARATION DE L'EXEMPLE 63 : 2-oxo 3-(difluorométhyl) 5-thiazol méthanol et produit 4,5-dihydro correspondant.

On chauffe à 80°C du 2-oxo 5-thiazol carboxylate de méthyle dans du diméthylformamide en présence de carbonate de potassium, traite par du Fréon 22 afin d'obtenir le 2-oxo 3-(difluorométhyl) 5-thiazol carboxylate de méthyle puis par du borohydrure de sodium en présence de méthanol pour obtenir l'alcool attendu.

### EXEMPLE 64 : 1R[1alpha,3alpha(E)] 3-(2-fluoro 3-méthoxy 3-oxo 1-propényl) 2,2-diméthyl cyclopropane carboxylate de [2-(pentafluoroéthyl) 4-thiazolyl) méthyle.

Rf = 0,11 (heptane-AcOEt 8-2).

### EXEMPLE 65 : 1R[1alpha,3alpha(E)] 3-(2-fluoro 3-méthoxy 3-oxo 1-propényl) 2,2-diméthyl cyclopropane carboxylate de (3-difluorométhyl 2-oxo 5-thiazolyl) méthyle.

### EXEMPLE 66 : 1R[1alpha,3alpha(E)] 3-(2-fluoro 3-méthoxy 3-oxo 1-propényl) 2,2-diméthyl cyclopropane carboxylate de (2-oxo 3-difluorométhyl 4,5-dihydro-(3H)-5-thiazolyl) méthyle.

Rf = 0,15 (hexane-AcOEt 8-2).

### EXEMPLE 67 : 1R[1alpha,3alpha(Z)] 2,2-diméthyl 3-(3,3,3-trifluoro 2-chloropropényl) cyclopropane carboxylate de 1-(3-difluorométhyl) 2-oxo 5-thiazolyl) 2-pyridyle.

Rf = 0,09 (heptane-CH₂Cl₂ 5-5).

### PREPARATION DE L'EXEMPLE 67 : 2-oxo 3-(difluorométhyl) alpha-éthynyl 5-thiazol méthanol.

En opérant comme à la préparation 14 à partir de l'alcool obtenu à la préparation 63, on obtient l'alcool attendu.

### EXEMPLE 68 : 1R[1alpha,3alpha(Z)] 2,2-diméthyl 3-(3,3,3-trifluoro 2-chloropropényl) cyclopropane carboxylate de (2-trifluorométhyl) 5-thiazolyl) méthyle.

Rf = 0,19 (heptane-CH₂Cl₂ 1-1).

### EXEMPLE 69 : 1R[1alpha,3alpha(Z)] 2,2-diméthyl 3-(3,3,3-trifluoro 2-chloropropényl) cyclopropane carboxylate de 1-(2-trifluorométhyl) 5-thiazolyl) 2-pyridinyle.

Rf = 0,2 (heptane-CH₂Cl₂ 5-5).

### PREPARATION DE L'EXEMPLE 69 : 2-trifluorométhyl alpha-éthynyl 5-thiazol méthanol.

En opérant comme à la préparation 1 à partir du 2-trifluorométhyl 5-thiazol carboxaldéhyde, on obtient l'alcool attendu.

### EXEMPLE 70 : 1R[1alpha,3alpha(Z)] 2,2-diméthyl 3-(3,3,3-trifluoro 2-chloropropényl) cyclopropane carboxylate de (2-difluorométhylthio) 4-thiazolyl) méthyle.

Rf = 0,14 (heptane-CH₂Cl₂ 5-5).

### EXEMPLE 71 : 1R[1alpha,3alpha] 3-(2,2-dichloroéthényl) 2,2-diméthyl cyclopropane carboxylate de 1-(2-trifluorométhyl 4-thiazolyl) 2-propynyle.

Rf = 0,14 (heptane-CH₂Cl₂ 5-5).

### EXEMPLE 72 : 1R[1alpha,3alpha(Z+E)] 2-chloro 2-fluoroéthényl) 2,2-diméthyl cyclopropane carboxylate de 1-(2-trifluorométhyl 4-thiazolyl) 2-propynyle.

Rf = 0,11 (heptane-CH₂Cl₂ 5-5).

### EXEMPLE 73 : 1R[1alpha,3alpha(Z)] 2,2-diméthyl 3-(3,3,3-trifluoro 2-chloropropényl) cyclopropane carboxylate de (2-trifluorométhyl 4-thiazolyl) propényle.

### EXEMPLE 74 : 1R[1alpha,3alpha(Z)] 3-(2-méthyl 1-propényl) 2,2-diméthyl cyclopropane carboxylate de 1-(2-trifluorométhyl 4-thiazolyl) 2-propynyle.

Rf = 0,24 (heptane-tBuOCH₃ 9-1).

### EXEMPLE 75 : 1R[1alpha,3alpha] 3-(2,2-difluoroéthényl) 2,2-diméthyl cyclopropane carboxylate de 1-(2-trifluorométhyl 4-thiazolyl) 2-propynyle.

Rf = 0,22 (heptane-tBuOCH₃ 9-1).

### EXEMPLE 76 : 1R[1alpha,3bêta(Z)] 3-(2-fluoro 3-méthoxy 3-oxo 1-propényl) 2,2-diméthyl cyclopropane carboxylate de 1-(2-trifluorométhyl 4-thiazolyl) 2-propynyle.

Rf = 0,23 (heptane-AcOEt 7-3).

### EXEMPLE 77 : 1R[1alpha,3alpha(Z)] 3-(3-oxo 3-[2,2,2-trifluoro-1-(trifluorométhyl) 1-propényl] 2,2-diméthyl cyclopropane carboxylate de 1-(2-trifluorométhyl 4-thiazolyl) 2-propynyle.

Rf = 0,19 (heptane-CH₂Cl₂ 5-5).

### EXEMPLE 78 : 1R[1alpha,3alpha(E)] 3-(2-fluoro 3-méthoxy 3-oxo 1-propényl) 2,2-diméthyl cyclopropane carboxylate de (2-trifluorométhyl 5-thiazolyl) méthyle.

Rf = 0,24 (heptane-AcOEt 7-3).

### EXEMPLE 79 : 1R[1alpha,3alpha(Z)] 2,2-diméthyl 3-(3,3,3-trifluoro 2-chloropropényl) cyclopropane carboxylate de 1-[2-(1-méthyléthyl) 4-thiazolyl] 2-propynyle.

Rf = 0,26 (heptane-AcOEt 9-1).

### PREPARATION DE L'EXEMPLE 79 : 2-(méthyléthyl) alpha-éthynyl 4-thiazol méthanol.

On opère comme à la préparation 56 à partir du thioisobutyramide pour obtenir le 2-(méthyléthyl) 4-thiazolyl carboxylate d'éthyle sur lequel on fait réagir le méthanol en présence de borohydrure de sodium pour obtenir le 2-(méthyléthyl) 4-thiazol méthanol que l'on traite par l'oxyde de manganèse pour obtenir l'aldéhyde correspondant puis opère comme la préparation 14 stade B pour obtenir l'alcool attendu.

### EXEMPLE 80 : 1R[1alpha,3alpha(E)] 3-(2-fluoro 3-méthoxy 3-oxo 1-propényl) 2,2-diméthyl cyclopropane carboxylate de 1-(2-difluorométhoxy 4-thiazolyl) 2-propynyle.

Rf = 0,10 (heptane-ether iso 9-1).

### EXEMPLE 81 : 1R[1alpha,3alpha(E)] 3-(2-fluoro 3-méthoxy 3-oxo 1-propényl) 2,2-diméthyl cyclopropane carboxylate de 1-(2-méthyléthyl 4-thiazolyl) 2-propynyle.

Rf = 0,20 (heptane-AcOEt 8-2).

### EXEMPLE 82 : 1R[1alpha,3alpha] 3-(2,2-dichloroéthényl) 2,2-diméthyl cyclopropane carboxylate de 1-(2-difluorométhoxy 4-thiazolyl) 2-propynyle.

Rf = 0,19 (heptane-CH₂Cl₂ 1-1).

### EXEMPLE 83 : 1R[1alpha,3alpha] 3-(2,2-difluoroéthényl) 2,2-diméthyl cyclopropane carboxylate de 1-(2-difluorométhoxy 4-thiazolyl) 2-propynyle.

Rf = 0,22 (heptane-CH₂Cl₂ 1-1).

### EXEMPLE 84 : 1R[1alpha,3alpha(Z+E)] 3-(2-chloro 2-fluoro éthényl) 2,2-diméthyl cyclopropane carboxylate de 1-(2-difluorométhoxy 4-thiazolyl) 2-propynyle.

Rf = 0,22 (CH₂Cl₂-heptane 1-1).

### EXEMPLE 85 : 1R[1alpha,3alpha(Z)] 3-(2-chloro 3,3,3-trichloro 1-propényl) 2,2-diméthyl cyclopropane carboxylate de 1-(5-bromo 4-trifluorométhyl 2-thiazolyl) éthyle.

Rf = 0,31 (heptane-CH₂Cl₂ 1-1).

### EXEMPLE 86 : 1R[1alpha,3alpha(Z)] 3-(2-chloro 3,3,3-trichloro 1-propényl) 2,2-diméthyl cyclopropane carboxylate de 1-(5-bromo 4-trifluorométhyl 2-thiazolyl) méthyle.

Rf = 0,26 (heptane-CH₂Cl₂ 1-1).

### EXEMPLE 87 : Préparation d'un concentré soluble

On effectue un mélange homogène de :

| | |
|---|---|
| Produit de l'exemple 1 : | 0,25 g |
| Butoxyde pipéronyle : | 1,00 g |
| Tween 80 : | 0,25 g |
| Topanol A : | 0,1 g |
| Eau : | 98,4 g |

### EXEMPLE 88 : Préparation d'un concentré émulsifiable

On mélange intimement :

| | |
|---|---|
| Produit de l'exemple 1 (isomère B) : | 0,015 g |
| Butoxyde de pipéronyle : | 0,5 g |
| Topanol A : | 0,1 g |
| Tween 80 : | 3,5 g |
| Xylène : | 95,885 g |

### EXEMPLE 89 : Préparation d'un concentré émulsifiable

On effectue un mélange homogène de :

| | |
|---|---|
| Produit de l'exemple 1 : | 1,5 g |
| Tween 80 : | 20,00 g |
| Topanol A : | 0,1 g |
| Xylène : | 78,4 g |

### EXEMPLE 90 : Préparation de granulés

On a préparé des granulés contenant de 0,1 % à 5 % de substances actives.

### ETUDE BIOLOGIQUE

A - Activité sur Diabrotica
   Les insectes tests sont des larves de dernier stade de Diabrotica.
   On traite une rondelle de papier filtre de 9 cm de diamètre, déposée au fond d'une boîte de Pétri, à l'aide de 2 cm³ de solution acétonique du produit à tester. Après séchage on dépose 10 larves par dose et on effectue le contrôle de mortalité 24 heures après le traitement.
   Dès la dose de 5 ppm les produits de l'invention présentent une bonne activité, notamment les produits des exemples 1, 62, 71, 72, 75, 83 et 84.
B - Etude de l'effet d'abattage sur mouche domestique
   Les insectes tests sont des mouches domestiques femelles âgées de 4 jours. On opère par pulvérisation en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.
   A la dose de 1 g/l les produits de l'invention présentent une bonne activité.
C - Etude de l'effet létal sur mouche domestique
   Les insectes tests sont des mouches domestiques femelles âgées de 4 à 5 jours. On opère par application topique de 1 microlitre de solution acétonique du produit à tester sur le thorax dorsal des insectes à l'aide du micro manipulateur d'Arnold. On utilise 50 individus par traitement. On effectue le contrôle de mortalité vingt-quatre heures après traitement.
   A la dose de 10 mg/l le produit de l'exemple 1 présente une bonne activité.

## Revendications

**1)** Sous toutes leurs formes stéréoisomères possibles, ainsi que leurs mélanges, les composés de formule (I) : 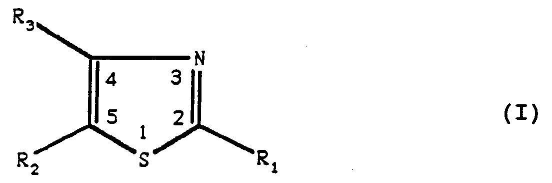 dans laquelle l'un des radicaux R₁, R₂ ou R₃ représente un radical : 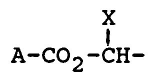 dans lequel :
X représente un atome d'hydrogène, un radical C≡N, un radical alkyle, alkényle ou alkynyle renfermant jusqu'à 4 atomes de carbone et
ou bien A représente un radical : 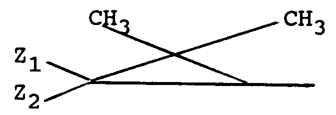 dans lequel Z₁ représente un atome d'hydrogène et
- soit Z₂ représente un radical : 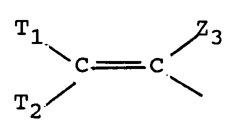 dans lequel Z₃ représente un atome d'hydrogène ou d'halogène et T₁ représente un atome d'hydrogène, un atome d'halogène, un radical alkyloxy ou alkyle renfermant de 1 à 8 atomes de carbone éventuellement substitués par des halogènes, un radical mono-, di- ou trifluorométhyle ou cyano ou un noyau phényle éventuellement substitué par un halogène et T₂ représente un atome d'hydrogène, un atome d'halogène, un radical alkyloxy renfermant de 1 à 8 atomes de carbone éventuellement substitué par des halogènes ou un radical alkyle renfermant de 1 à 8 atomes de carbone substitués par des halogènes, un radical mono-, di- ou trifluorométhyle ou cyano ou un noyau phényle éventuellement substitué par un halogène ou T₁ et T₂ forment ensemble un radical cycloalkyle renfermant de 3 à 6 atomes de carbone ou un radical : 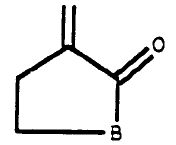 dans lequel B représente un atome d'oxygène ou de soufre ;
- soit Z₂ représente un radical : 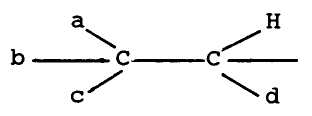 dans lequel a, b, c et d, identiques ou différents représentent chacun un atome d'halogène,
- soit Z₂ représente un radical : 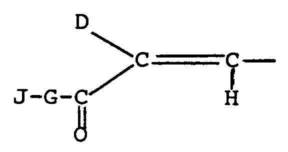 dans lequel D représente un atome d'hydrogène ou d'halogène, un radical alkyloxy renfermant de 1 à 8 atomes de carbone, G représente un atome d'oxygène ou de soufre et J représente ou bien un radical alkyle linéaire, ramifié ou cyclique, saturé ou insaturé, renfermant de 1 à 8 atomes de carbone, éventuellement substitué par un ou plusieurs groupements fonctionnels, identiques ou différents, ou bien un groupement aryle renfermant de 6 à 14 atomes de carbone, éventuellement substitué par un ou plusieurs groupements fonctionnels identiques ou différents, ou bien un radical hétérocyclique éventuellement substitué par un ou plusieurs groupements fonctionnels, identiques ou différents,
ou bien A représente un radical : 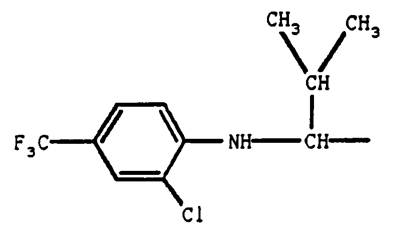 ou un radical : 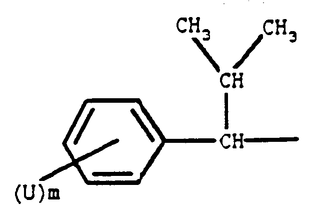 dans lequel U, en position quelconque sur le noyau benzénique, représente un atome d'halogène, un radical alkyle renfermant de 1 à 8 atomes de carbone ou un radical alcoxy renfermant de 1 à 8 atomes de carbone, m représentant le nombre 0, 1 ou 2 et quand m est 2, les substituants U peuvent être identiques ou différents, et les deux radicaux choisis parmi les radicaux R₁, R₂et R₃ qui ne représentent pas un radical : 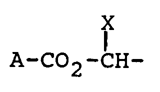 sont identiques ou différents et représentent ou un atome d'hydrogène ou un atome d'halogène ou un radical alkyle, alkényle ou alkynyle renfermant jusqu'à 8 atomes de carbone éventuellement substitué par un ou plusieurs atomes d'halogène ou un radical hydroxyle, O-alkyle, O-alkényle ou O-alkynyle, CO₂-alkyle, CO₂-alkényle, CO₂-alkynyle, S(O)ₙ-alkyle, S(O)ₙ-alkényle ou S(O)ₙ-alkynyle, n représentant le nombre 0, 1 ou 2, renfermant jusqu'à 8 atomes de carbone, éventuellement substitué par un ou plusieurs atomes d'halogène ou un radical aryle, O-aryle ou thioaryle renfermant jusqu'à 14 atomes de carbone éventuellement substitué par un ou plusieurs radicaux choisis dans le groupe constitué par les atomes d'halogène, les radicaux alkyle, alkényle ou alkynyle renfermant jusqu'à 8 atomes de carbone éventuellement substitué par un ou plusieurs atomes d'halogène et les radicaux hydroxy libres estérifiés ou éthérifiés ou un radical hétéroaryle ou hétéroaryloxy ou un radical C≡N, NH₂ ou NO₂.

**2)** Les composés de formule (I) dans lesquels l'un des radicaux R₁, R₂ et R₃ représente un atome d'hydrogène.

**3)** Les composés de formule (I) tels que définis à la revendication 1 ou 2 dans lesquels celui des radicaux R₁, R₂ ou R₃ qui représente un radical : 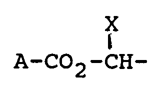 est situé en position 4.

**4)** Les composés de formule (I) tels que définis à la revendication 3 dans lesquels l'un des radicaux R₁, R₂ ou R₃ représente un atome d'hydrogène en position 5.

**5)** Les composés de formule (I) tels que définis à l'une quelconque des revendications 1 à 4 dans lesquels l'un des radicaux R₁, R₂ ou R₃ représente un radical alkyle renfermant jusqu'à 4 atomes de carbone éventuellement substitué par un ou plusieurs atomes d'halogène.

**6)** Les composés de formule (I) tels que définis à l'un quelconque des revendications 1 à 5 dans lesquels l'un des radicaux R₁, R₂ ou R₃ représente un radical O-alkyle ou S-alkyle, éventuellement substitué par un ou plusieurs atomes d'halogène.

**7)** Les composés de formule (I) tels que définis à l'une des revendications 5 ou 6 dans lesquels le radical alkyle, O-alkyle, ou S-alkyle est substitué par un ou plusieurs atomes de fluor.

**8)** Les composés de formule (I) tels que définis à la revendication 5 dans lesquels l'un des radicaux R₁, R₂ ou R₃ représente un radical CF₃.

**9)** Les composés de formule (I) tels que définis à la revendication 6 dans lesquels l'un des radicaux R₁, R₂ ou R₃ représente un radical OCHF₂.

**10)** Les composés de formule (I) tels que définis à l'une quelconque des revendications 1 à 9 dans lesquels X représente un radical éthynyle.

**11)** Les composés de formule (I) tels que définis à l'une quelconque des revendications 1 à 9 dans lesquels X représente un atome d'hydrogène.

**12)** Les composés de formule (I) tels que définis à l'une quelconque des revendications 1 à 11 dans lesquels l'un des radicaux R₁, R₂ ou R₃ représente un radical :

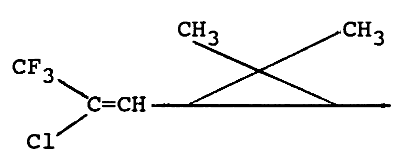

**13)** Le composé de formule (I) tel que défini à la revendication 1 dont le nom suit :
- le [1R-[1alpha, 3alpha(Z)]]-3-(2-chloro 3,3,3-trifluoro 1-propényl) 2,2-diméthyl cyclopropanecarboxylate de 1-[2-(trifluorométhyl) 4-thiazolyl] 2-propynyle.

**14)** Procédé de préparation des composés de formule (I) tels que définis à l'une quelconque des revendications 1 à 13, caractérisé en ce que l'on soumet un acide de formule (II) :
ACO₂H (II)
A étant défini comme précédemment, ou un dérivé fonctionnel de cet acide, à l'action d'un alcool de formule (III) : 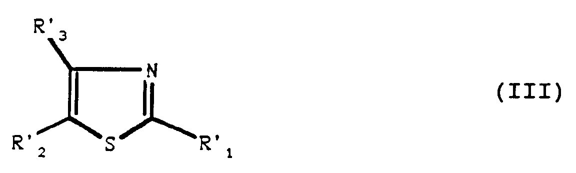 dans lequel l'un des radicaux R'₁, R'₂ ou R'₃ représente un radical : 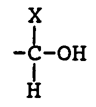 X conservant sa signification précécente et les deux autres radicaux ont la même signification que les radicaux R₁, R₂ et R₃ à l'exception de : 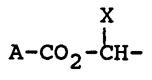 ou d'un dérivé fonctionnel de cet alcool de formule (III) pour obtenir le composé de formule (I) correspondant.

**15)** A titre de produits chimiques nouveaux les composés de formule (III) dont les noms suivent :
- 2-(1-fluoro 2-propynyl) 4-thiazol méthanol
- 2-bromo 4-thiazol méthanol
- 2-bromo alpha-éthynyl 4-thiazol méthanol
- 1-[2-(trifluorométhyl) 4-thiazolyl] éthanol
- alpha-éthynyl 2-méthyl 4-thiazol méthanol
- 2-fluoro 4-thiazol méthanol
- 1-[2-(difluorométhoxy) 4-thiazolyl] éthanol
- 2-(difluorométhoxy) alpha-éthynyl 4-thiazol méthanol
- 2-(difluorométhoxy) 4-thiazol méthanol
- alpha-éthynyl 2-(trifluorométhyl) 4-thiazol méthanol
- 1-(2-chloro 4-thiazolyl) éthanol
- 2-chloro alpha-éthynyl 4-thiazol méthanol
- 2-(méthylthio) 4-thiazol méthanol
- 2-(méthylthio) alpha-éthynyl 4-thiazol méthanol
- 2-fluoro alpha-éthynyl 4-thiazol méthanol
- 2-éthynyl 4-thiazol méthanol
- 2-éthynyl alpha-éthynyl 4-thiazol méthanol
- 2-pentafluoroéthyl 4-thiazol méthanol
- 2-pentafluoroéthyl alpha-éthynyl 4-thiazol méthanol
- 2-(difluoroéthyl) alpha-éthynyl 4-thiazol méthanol
- 2-oxo 3-(difluorométhyl) 5-thiazol méthanol
- 2-oxo 3-(difluorométhyl 4,5-dihydro-(3H)-5-thiazol méthanol
- 2-oxo 3-(dilfuorométhyl) alpha-éthynyl 5-thiazol méthanol
- 2-tridluorométhyl alpha-éthynyl 5-thiazol méthanol
- 2-(méthyléthyl) 4-thiazol méthanol
- 2-méthyléthyl) alpha-éthynyl 4-thiazol méthanol.

**16)** Application des composés de formule (I) tels que définis à l'une quelconque des revendications 1 à 13, à la lutte contre les parasites des végétaux et les parasites des locaux.

**17)** Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 13.

**18)** Les compositions insecticides, caractérisées en ce qu'elles renferment comme principe actif au moins un composé défini à l'une quelconque des revendications 1 à 13.

**19)** Les compositions insecticides définies à la revendication 18 caractérisées en ce qu'elles sont destinées à la lutte contre DIABROTICA et les autres parasites du sol.

**20)** Les compositions acaricides, caractérisées en ce qu'elles renferment comme principe actif au moins un composé défini à l'une quelconque des revendications 1 à 13.

**21)** Associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alphacyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.
